(19) Europäisches Patentamt
European Patent Office
Office européen des brevets

(11) Veröffentlichungsnummer: **0 052 817**
**A1**

(12) # EUROPÄISCHE PATENTANMELDUNG

(21) Anmeldenummer: **81109478.8**

(22) Anmeldetag: **02.11.81**

(51) Int. Cl.³: **C 07 C 85/24**
C 07 C 87/60

(30) Priorität: **15.11.80 DE 3043206**

(43) Veröffentlichungstag der Anmeldung:
**02.06.82 Patentblatt 82/22**

(84) Benannte Vertragsstaaten:
**BE CH DE FR GB LI**

(71) Anmelder: **BAYER AG**
**Zentralbereich Patente, Marken und Lizenzen**
**D-5090 Leverkusen 1, Bayerwerk(DE)**

(72) Erfinder: **Schnegg, Ulrich, Dr.**
**Ulrich-von-Hassell-Strasse 13**
**D-5090 Leverkusen 1(DE)**

(72) Erfinder: **Wedemeyer, Karlfried, Dr.**
**Bilharzstrasse 7**
**D-5000 Köln 80(DE)**

(54) **Verfahren zur Herstellung von 4-Chlor-2,6-dialkylanilinen.**

(57) Die Erfindung betrifft ein Verfahren zur Herstellung von 4-Chlor-2,6-dialkylanilinen durch Chlorierung der Ammoniumsalze der 2,6-Dialkylaniline in Gegenwart eines inerten, organischen Lösungs- und/oder Verdünnungsmittels und gegebenenfalls in Gegenwart von Friedel-Crafts-Katalysatoren und anschließender Hydrolyse des Chlorierungsgemisches.

Die erfindungsgemäß hergestellten 4-Chlor-2,6-dialkylaniline finden als Zwischenprodukte für Pflanzenschutzmittel Verwendung.

EP 0 052 817 A1

Croydon Printing Company Ltd.

BAYER AKTIENGESELLSCHAFT 5090 Leverkusen, Bayerwerk

Zentralbereich
Patente, Marken und Lizenzen Bg/bc/kl-c 14.11.80

# Verfahren zur Herstellung von 4-Chlor-2,6-dialkylanilinen

Die Erfindung betrifft ein Verfahren zur Herstellung von 4-Chlor-2,6-dialkylanilinen durch Chlorierung der entsprechenden 2,6-Dialkylaniline.

Aus der US-PS 2 675 409 ist ein Verfahren zur Herstellung von 2,4,6-Trichloranilinen bekannt, bei dem man die entsprechenden Anilin-Hydrochloride in einem inerten, organischen Lösungsmittelsystem mit überschüssigem Chlor umsetzt.

Ein ähnliches Verfahren ist in der DE-OS 2 319 645 beschrieben. Gemäß dieser Offenlegungsschrift wird 5-Chlor-2-toluidin durch Umsetzung des in Halogenkohlenwasserstoffen suspendierten o-Toluidinhydrochlorids mit Chlor hergestellt.

In der DE-OS 2 916 714 ist auf Seite 5, zweitletzter Absatz, erwähnt, daß man 4-Chlor-2,6-dialkylaniline durch Chlorierung der entsprechenden Anilinverbindungen herstellen kann. Genauere Einzelheiten über die Chlorierung sowie die dabei erhaltenen Ausbeuten an Chlorierungsprodukten sind allerdings der DE-OS 2 916 714 nicht zu entnehmen.

Die Herstellung von 4-Chlor-2,6-dimethylanilin durch direkte Chlorierung von 2,6-Dimethylanilin in Eisessig ist in J.Chem.Soc. 1927, Seite 1106, 1107 beschrieben. Dabei werden jedoch nur schlechte Ausbeuten an Chlorierungsprodukt erhalten, weshalb die Autoren darauf hinweisen, daß die direkte Chlorierung von 2,6-Dimethylanilin nicht sehr geeignet ist zur Herstellung von 4-Chlor-2,6-dimethylanilin.

Neben dem Nachteil der schlechten Ausbeuten beinhaltet das in J.Chem.Soc. 1927, Seite 1106, 1107 beschriebene Verfahren noch weitere Nachteile. Da die Chlorierung in Eisessig durchgeführt wird, ist es zur Isolierung des Chlorierungsproduktes erforderlich, den Eisessig mit Wasser zu verdünnen und die essigsaure Lösung mit Alkali zu neutralisieren. Das dabei anfallende Salz der Essigsäure, z.B. Natriumacetat, das nur schwer abzutrennen ist, gelangt daher ins Abwasser, wo es nicht nur die Salzfracht, sondern auch den chemischen und biologischen Sauerstoffbedarf stark erhöht.

Es wurde nun ein Verfahren zur Herstellung von 4-Chlor-2,6-dialkylanilinen gefunden, das dadurch gekennzeichnet ist, daß man die Ammoniumsalze der 2,6-Dialkylaniline mit einem Chlorierungsmittel bei -15 bis 100°C in Gegenwart eines inerten, organischen Lösungs- und/oder Verdünnungsmittels und gegebenenfalls

in Gegenwart von Friedel-Crafts-Katalysatoren umsetzt und anschließend aus dem erhaltenen 4-Chlor-2,6-dialkylaniliniumsalz 4-Chlor-2,6-dialkylanilin freisetzt.

Als 2,6-Dialkylaniline können solche der Formel

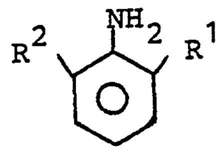 ,

worin

$R^1$ und $R^2$ gleich oder verschieden sind und für einen Alkylrest mit 1 bis 4, bevorzugt 1 oder 2, Kohlenstoffatomen stehen,

in das erfindungsgemäße Verfahren eingesetzt werden.

Zum Beispiel können folgende 2,6-Dialkylaniline in das erfindungsgemäße Verfahren eingesetzt werden: 2,6-Dimethylanilin, 2-Ethyl-6-methylanilin, 2-Isopropyl-6-methylanilin, 2,6-Diethylanilin, 2-Ethyl-6-n-propylanilin, 2-n-propyl-6-methylanilin und 2-Ethyl-6-isopropylanilin.

Bevorzugt werden 2,6-Dimethylanilin, 2,6-Diethylanilin und 2-Ethyl-6-methylanilin eingesetzt.

Bei der Durchführung des erfindungsgemäßen Verfahrens geht man im allgemeinen so vor, daß man das 2,6-Dialkylanilin in einem inerten, organischen Lösungs- und/oder Verdünnungsmittel löst bzw. suspendiert und dann mit Protonensäuren, wie Chlorwasserstoffsäure, Schwefelsäure oder Phosphorsäure, vorzugsweise Chlorwasserstoffsäure, das 2,6-Dialkylaniliniumsalz ausfällt.

Selbstverständlich ist es auch möglich, das 2,6-Dialkylanilin in einem inerten,organischen Lösungs- und/oder Verdünnungsmittel mit wäßriger Säure zu versetzen und dann das Wasser, z.B. durch Azeotropdestillation, abzudestillieren. Eine weitere Methode, die Ammoniumsalze der 2,6-Dialkylaniline herzustellen, besteht z.B. darin, daß man das Salz unabhängig vom für die Chlorierung verwendeten Lösungs- und/oder Verdünnungsmittel in einem beliebigen Lösungs- und/oder Verdünnungsmittel, z.B. in Wasser, mit Protonensäure herstellt, das erhaltene 2,6-Dialkylanilinium-Salz abtrennt und als solches in das erfindungsgemäße Verfahren einsetzt.

Als inerte, organische Lösungs- und/oder Verdünnungsmittel werden üblicherweise solche in das erfindungsgemäße Verfahren eingesetzt, in denen die Löslichkeit des 2,6-Dialkylanilinium-Salzes gering ist, so daß die Hauptmenge des Salzes in kristalliner Form als Suspension vorliegt. Da die Selektivität und die Ausbeuten bei der Chlorierung umso größer sind, je kleiner die Löslichkeit des Aniliniumsalzes im verwendeten Lösungs- und/oder Verdünnungsmittel ist, andererseits aber die Reaktionsgeschwindigkeit mit der Löslichkeit

des Aniliniumsalzes zunimmt, ist es zweckmäßig, einen technisch sinnvollen Kompromiß zwischen höchster Selektivität und größter Raum/Zeit-Ausbeute zu finden. Um ein Optimum an Selektivität und Raum/Zeit-Ausbeute zu erreichen, kann man so vorgehen, daß man einem unpolaren, schlecht lösenden organischen Lösungsmittel einen Lösungsvermittler, der oft aus einem polaren organischen Lösungsmittel besteht, zufügt. Da die optimale Löslichkeit des Aniliniumsalzes in dem verwendeten inerten, organischen Lösungs- und/oder Verdünnungsmittel von verschiedenen Faktoren, wie den beiden Alkylresten des Dialkylanilins und der Art der Protonensäure sowie deren Löslichkeit im Lösungs- und/oder Verdünnungsmittel, abhängt, ist es zweckmäßig, vor der eigentlichen Umsetzung, das für die Chlorierung am besten geeignete Lösungs- und/oder Verdünnungsmittel durch leicht durchzuführende Vorversuche zu ermitteln.

Als inerte, organische Lösungs- und/oder Verdünnungsmittel können aromatische und aliphatische Kohlenwasserstoffe oder aliphatische Halogenkohlenwasserstoffe mit 1 bis 15, bevorzugt 1 bis 8, Kohlenstoffatomen, wie Methylenchlorid, Tetrachlorkohlenstoff, Trichlorethan, Hexan, Isooctan, Benzol, Toluol oder Xylol, bevorzugt Tetrachlorkohlenstoff, Isooctan oder Toluol, in das erfindungsgemäße Verfahren eingesetzt werden.

Weiterhin können die inerten, organischen Lösungs- und/oder Verdünnungsmittel noch Protonensäuren, wie Chlorwasserstoffsäure, Schwefelsäure oder Phosphorsäure, bis zur Sättigung gelöst, enthalten.

Zur Erhöhung der Löslichkeit der Aniliniumsalze in den verwendeten Lösungs- und/oder Verdünnungsmitteln können niedere Alkohole, wie Methanol, Ethanol oder Propanol niedere Carbonsäuren und Carbonsäurederivate, wie Essigsäure oder Dimethylformamid, und/oder cyclische Ether, wie Tetrahydrofuran oder Dioxan, in das erfindungsgemäße Verfahren eingesetzt werden.

Die Menge der einzusetzenden Lösungs- und/oder Verdünnungsmittel, die sowohl einzeln als auch im Gemisch untereinander eingesetzt werden können, können in weiten Bereichen schwanken. Im allgemeinen werden etwa 500 bis 3000 ml, bevorzugt 750 bis 1500 ml, bezogen auf 1 Mol Dialkylanilin, eingesetzt.

Als Chlorierungsmittel werden üblicherweise Chlorgas oder Chlorgas enthaltende Gasgemische, wie ein Gemisch aus Chlor und Luft, Chlor und Kohlendioxid, Chlor und Chlorwasserstoff oder Chlor und Stickstoff, verwendet. Es ist jedoch auch möglich, andere Chlorierungsmittel, wie Sulfurylchlorid, in das erfindungsgemäße Verfahren einzusetzen.

Bei Einsatz von Chlorgas oder Chlorgas enthaltenden

Gasgemischen setzt man im allgemeinen 0,8 bis 1,8, bevorzugt 1,0 bis 1,5, Mol Chlor pro Mol Ausgangsprodukt ein.

Wird ein anderes Chlorierungsmittel, wie Sulfurylchlorid, verwendet, beträgt die Menge an einzusetzendem Chlorierungsmittel etwa 0,8 bis 1,5, bevorzugt 0,9 bis 1,2, Mol Chlorierungsmittel pro Mol Einsatzprodukt.

Bei der Durchführung der Chlorierungsreaktion kann es vorteilhaft sein, in Gegenwart von üblichen Friedel-Crafts-Katalysatoren, wie $FeCl_3$, $AlCl_3$ und/oder Jod, zu arbeiten.

Die Menge der Friedel-Crafts-Katalysatoren ist dabei nicht kritisch. Man setzt im allgemeinen etwa 0,1 bis 10 Mol-%, bevorzugt 0,5 bis 5 Mol-%, bezogen auf Dialkylanilin ein.

Das erfindungsgemäße Verfahren wird üblicherweise bei etwa -15 bis 100°C, bevorzugt bei 0 bis 80°C, durchgeführt.

Das Verfahren kann sowohl kontinuierlich als auch diskontinuierlich bei Normaldruck als auch bei erhöhtem Druck (ca. 1,0 bis 1,5 bar) durchgeführt werden.

Nach Beendigung der Chlorierungsreaktion können die während der Chlorierung der 2,6-Dialkylaniline gebildeten Nebenprodukte in an sich bekannter Weise aus dem Reaktionsgemisch entfernt werden. So kann man bei der Verwendung von Tetrachlorkohlenstoff als Lösungs- und/oder Verdünnungsmittel das Reaktionsgemisch einer Hydrolyse mit Wasser oder wäßriger Säure unterwerfen. Die Aniliniumsalze können dann, evtl. nach Entfernen des Wassers, auf geeignete Weise, beispielsweise durch Destillation, aus der Suspension abgetrennt und mit Alkali in wäßrigem Medium die Aniline freigesetzt werden. Nach Abtrennen der Salzlösung kann das rohe 4-Chlor-2,6-dialkylanilin durch Destillation weiter gereinigt werden. Bei dieser Aufarbeitungsweise verbleiben die hydrolysierbaren Nebenprodukte in der Tetrachlorkohlenstoff-Phase.

Gemäß einer anderen Variante ist es auch möglich, das Chlorierungsgemisch mit verdünnter Säure zu extrahieren, wobei die Aniliniumsalze in die wäßrige Phase übergehen, aus der mit Alkali das rohe 4-Chlor-2,6-dialkylanilin freigesetzt wird.

Weiterhin ist es möglich, dem Reaktionsgemisch Wasser oder eine wäßrige Säure zuzugeben und dann das Lösungs- und/oder Verdünnungsmittel und das rohe 4-Chlor-2,6-Dialkylanilin durch Wasserdampf überzutreiben.

Die Hydrolyse des Rohgemisches dient bei dem erfindungsgemäßen Verfahren der Betriebssicherheit, da sich gebildete, hydrolysierbare Nebenprodukte unter den thermischen Bedingungen einer Destillation leicht zersetzen.

Nach dem erfindungsgemäßen Verfahren werden die 4-Chlor-2,6-dialkylaniline in guten Ausbeuten und in hoher Reinheit erhalten. Dabei ist es außerordentlich überraschend, daß im Rohprodukt die Isomeren 3- und 5-Chlor-2,6-dialkylaniline nicht nachweisbar sind, wodurch die Aufarbeitung und Reinigung des Rohproduktes einfach und damit wirtschaftlich durchgeführt werden kann.

Es war nämlich zu erwarten, daß bei der Chlorierung der Aniliniumsalze neben dem gewünschten 4-Chlor-2,6-dialkylanilin in beträchtlichem Maße die Isomeren 3- und 5-Chlor-2,6-dialkylaniline sowie 3,4- und 4,5-Dichlor-2,6-dialkylaniline gebildet werden.

Auch im Hinblick auf die DE-OS 2 319 645, der gemäß o-Toluidinhydrochlorid mit Chlor in Gegenwart von Halogenkohlenwasserstoffen als Lösungsmittel zu 5-Chlor-2-toluidin umgesetzt wird, ist die selektive Chlorierung der 2,6-Dialkylaniline zu 4-Chlor-2,6-dialkylanilinen bei dem erfindungsgemäßen Verfahren als besonders überraschend zu werten. Danach war nämlich zu erwarten, daß die Chlorierung von 2,6-Dialkylanilin in Form seines Hydrochlorids das gleiche Isomere liefert wie die Chlorierung des entsprechenden Acetamids. Die Chlorierung von 2,6-Dialkylacetanilid

liefert aber 3-Chlor-2,6-dialkylacetanilid, wie eigene Untersuchungen ergeben haben. Auch das Ausbleiben des nach der oben aufgeführten Offenlegungsschrift zu erwartenden 3,4- und 4,5-Dichlor-2,6-dialkylanilins muß als besonders überraschend angesehen werden.

Die erfindungsgemäß hergestellten 4-Chlor-2,6-dialkylaniline finden als Zwischenprodukte für Pflanzenschutzmittel Verwendung (vgl. DE-OS 2 916 714).

Die nachfolgenden Beispiele sollen das erfindungsgemäße Verfahren verdeutlichen, ohne es jedoch auf diese Beispiele einzuschränken.

Beispiel 1

In einem 3 l Planschliffbecher werden 3 Mol 2,6-Dimethylanilin in 2300 g $CCl_4$ und 30 g Ethanol gelöst. Anschließend wird bei Raumtemperatur HCl-Gas bis zur Sättigung eingeleitet. Nach erfolgter Sättigung wird auf 10 bis 15°C abgekühlt und bei dieser Temperatur im Verlauf von 4 Stunden 270 g Chlor durch die entstandene Suspension durchgeleitet. Nach Beendigung der Chlorierung werden 250 ml 15 %ige Salzsäure zugegeben und dann die Salzsäure azeotrop wieder abdestilliert. Nach dem Abkühlen auf 20°C wird das rohe 4-Chlor-2,6-dimethylanilinhydrochlorid in Form von farblosen Kristallen abgenutscht. Aus dem Salz erhält man durch Aufnahme in Wasser und Neutralisation mit Natronlauge 331 g eines Anilingemisches, welches sich aus 14 % 2,6-Dimethylanilin und 84 % 4-Chlor-2,6-dimethylanilin zusammensetzt. Die Ausbeute, bezogen auf umgesetztes 2,6-Dimethylanilin, beträgt 69 %.

Aus dem Rohprodukt erhält man durch fraktionierte Destillation das Reinprodukt bei 115 bis 117°C/5 Torr, welches eine gaschromatographische Reinheit von 99 % und einen Schmelzpunkt von 42°C aufweist.

Beispiel 2

Beispiel 1 wird wiederholt, aber statt des Gemisches aus $CCl_4$/Ethanol wird Toluol als Lösungsmittel verwendet. Nach Extraktion des rohen Anilinhydrochlorids mit

verdünnter Salzsäure und Freisetzen der Base mit NaOH erhält man 357 g eines rohen 4-Chlor-2,6-dimethylanilins, welches neben 54 % 2,6-Dimethylanilin 46 % 4-Chlor-2,6-dimethylanilin enthält. Die Ausbeute, bezogen auf umgesetztes 2,6-Dimethylanilin, beträgt 75 %.

Beispiel 3

Analog Beispiel 1 werden 3 Mol 2-Ethyl-6-methylanilin in $CCl_4$ zuerst mit Salzsäure und dann mit Chlor begast. Nach der üblichen Aufarbeitung erhält man das Hydrochlorid des rohen 4-Chlor-2-ethyl-6-methylanilins in Form farbloser Kristalle, aus denen 334 g rohes Amingemisch freigesetzt werden. Ein Gaschromatogramm des rohen Amins zeigt 12 % 2-Ethyl-6-methylanilin neben 88 % 4-Chlor-2-ethyl-6-methylanilin an, entsprechend einer Ausbeute von 66 %, wenn man auf umgesetztes Ausgangsmaterial bezieht.

Aus dem rohen Produktgemisch wird bei einer Kopftemperatur von 139°C/13 Torr das gaschromatographisch reine 4-Chlor-2-ethyl-6-methylanilin gewonnen.

Beispiel 4

Beispiel 3 wird wiederholt, aber statt Tetrachlorkohlenstoff wird Toluol als Lösungsmittel verwendet und das Chlor bei 80°C eingeleitet. Nach üblicher Aufarbeitung erhält man 363 g Anilingemisch, welches 22 % Ausgangsmaterial neben 73 % 4-Chlor-2-ethyl-6-methylanilin enthält. Die Ausbeute auf umgesetztes Anilin bezogen, beträgt 64 %.

Beispiel 5

Beispiel 3 wird mit 2,6-Diethylanilin wiederholt, aber das Chlor in Gegenwart von 10 g Jod als Katalysator eingeleitet. Nach üblicher Aufarbeitung erhält man 279 g eines Amingemisches, welches laut Gaschromatogramm 39 % Diethylanilin neben 45 % 4-Chlor-2,6-diethylanilin enthält. Dies entspricht einer Ausbeute von 60 %, bezogen auf umgesetztes Diethylanilin.

Die Destillation liefert bei 102°C/0,2 Torr das gaschromatographisch reine Produkt.

Beispiel 6

Beispiel 1 wird wiederholt, aber unter Weglassung des Ethanols und Ersatz des Chlors durch Sulfurylchlorid als Chlorierungsmittel, welches bei einer Temperatur von 45 - 50°C zugegeben wird. Nach einer Wasserdampfdestillation des alkalisierten Rohproduktes erhält man 4-Chlor-2,6-dimethylanilin in 70 %iger Ausbeute.

Patentansprüche

1) Verfahren zur Herstellung von 4-Chlor-2,6-dialkylanilinen, dadurch gekennzeichnet, daß man die Ammoniumsalze der 2,6-Dialkylaniline mit einem Chlorierungsmittel bei -15 bis 100°C in Gegenwart eines inerten, organischen Lösungs- und/oder Verdünnungsmittels und gegebenenfalls in Gegenwart von Friedel-Crafts-Katalysatoren umsetzt und anschließend aus dem erhaltenen 4-Chlor-2,6-dialkylaniliniumsalz 4-Chlor-2,6-dialkylanilin freisetzt.

2) Verfahren nach Anspruch 1, dadurch gekennzeichnet, daß man das Chlorierungsgemisch einer Hydrolyse mit Wasser oder wäßriger Säure unterwirft.

3) Verfahren nach Ansprüchen 1 und 2, dadurch gekennzeichnet, daß man als Chlorierungsmittel Chlorgas, Chlorgas enthaltende Gasgemische oder Sulfurylchlorid einsetzt.

4) Verfahren nach Ansprüchen 1 bis 3, dadurch gekennzeichnet, daß man die Chlorierungsmittel in Mengen von 0,8 bis 1,5 Mol pro Mol Einsatzprodukt einsetzt.

5) Verfahren nach Ansprüchen 1 bis 4, dadurch gekennzeichnet, daß man als inerte, organische Lösungs- und/oder Verdünnungsmittel aromatische- und/oder aliphatische Kohlenwasserstoffe und/oder aliphatische Halogenkohlenwasserstoffe mit 1 bis 15 Kohlenstoffatomen einsetzt.

6) Verfahren nach Ansprüchen 1 bis 4, dadurch gekennzeichnet, daß man als inerte, organische Lösungs- und/oder Verdünnungsmittel Tetrachlorkohlenstoff, Isooctan und/oder Toluol einsetzt.

7) Verfahren nach Ansprüchen 1 bis 6, dadurch gekennzeichnet, daß man pro Mol Einsatzprodukt 500 bis 3000 ml eines inerten, organischen Lösungs- und/oder Verdünnungsmittels einsetzt.

8) Verfahren nach Ansprüchen 1 bis 7, dadurch gekennzeichnet, daß man als Friedel-Crafts-Katalysatoren $FeCl_3$, $AlCl_3$ und/oder Jod einsetzt.

9) Verfahren nach Ansprüchen 1 bis 8, dadurch gekennzeichnet, daß man die Friedel-Crafts-Katalysatoren in Mengen von 0,1 bis 10 Mol-%, bezogen auf das Einsatzprodukt, einsetzt.

10) Verfahren nach Ansprüchen 1 bis 9, dadurch gekennzeichnet, daß man die Umsetzung bei 0 bis 80°C durchführt.

Europäisches Patentamt

**0052817**

Nummer der Anmeldung

EP 81 10 9478

# EUROPÄISCHER RECHERCHENBERICHT

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| X | FR - A - 2 288 083 (HOECHST A.G.)<br>* Seite 1, Zeile 27 bis Seite 2, Zeile 13; Seite 3, Zeilen 12-20 *<br>--- | 1-10 | C 07 C 85/24<br>C 07 C 87/60 |
| X | DE - A - 2 337 621 (ASAHI DENKA KOGYO K.K.)<br>* Seiten 6-11; Beispiele *<br>--- | 1-10 | |
| A | ACTA CHEMICA SCANDINAVIA, Band 19, Heft 9, 1965, COPENHAGEN (DM)<br>H. HJEDS et al.: "Oximes, O-Methyloximes and N-Methyloximes of 2,6-Dimethyl-4-halogenobenzaldehyde", Seiten 2166-2174<br>* Seite 2171; "Experimental" *<br>------------ | 1 | RECHERCHIERTE SACHGEBIETE (Int. Cl.³)<br>C 07 C 85/00<br>C 07 C 87/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

x Der vorliegende Recherchenbericht wurde für alle Patentansprüche erstellt.

| Recherchenort | Abschlußdatum der Recherche | Prüfer |
|---|---|---|
| Den Haag | 09.02.1982 | PAUWELS |

EPA form 1503.1 06.78